# EUROPEAN PATENT SPECIFICATION

(11) **EP 3 449 833 B1**
(45) Date of publication and mention of the grant of the patent: **25.09.2019**
(21) Application number: 17188255.8
(22) Date of filing: 29.08.2017
(51) Int. Cl.: A61B 6/03, H01F 38/18, H04B 5/00

(54) **SPLITTABLE ROTARY JOINT MODULE WITH CONTACTLESS DATA LINK**
TEILBARES SCHLEIFRINGMODUL MIT KONTAKTLOSER DATENVERBINDUNG
MODULE DE JOINT ROTATIF SÉPARABLE AYANT UNE LIAISON DE DONNÉES SANS CONTACT

(43) Date of publication of application: 06.03.2019
(73) Proprietor: Schleifring GmbH, 82256 Fürstenfeldbruck (DE)
(72) Inventor: KNOBL, Horst, 86956 Schongau (DE); STAFFLER, Herbert, 82256 Fürstenfeldbruck (DE)
(74) Representative: Lohr, Jöstingmeier & Partner

(56) References cited:
- US-A- 5 600 697
- US-A1- 2013 187 740
- US-A1- 2015 265 225
- US-A1- 2016 211 822

## Description

### Field of the invention

The invention relates to rotary joints comprising capacitive couplers for noncontacting or contactless signal and data transmission. Such rotary joints may be used in computer tomography scanners, also called CT scanners.

### Description of the related art

Capacitive capacitive rotary joints are used to couple signals and data between parts rotating against each other. For example, in CT scanners, a rotating x-ray tube and an x-ray detector generate high-speed imaging data. The data may be transmitted from the rotating part to the stationary part. Furthermore, control signals for controlling the device and specifically the power supply of the x-ray tube may be transmitted from the stationary to the rotating part and vice versa. Many further applications exist where there is the need to transmit control signals or data between a rotor and a stator like in windmills, revolving transfer machines, bottling plants, packaging machines or placement heads of insertion machines.

A capacitive rotary joint for CT scanners is disclosed in US 5,600,697. A large diameter rotating ring carries a differentially driven strip line guiding a signal along this circumference of the ring. At the stationary side, there is a capacitive coupler picking up the signal from the near field of the strip line.

A bidirectional capacitive coupler is disclosed in US 2013/0214614. Here, the channels for the communication from the rotating to the stationary side and vice versa are interleaved.

A noise immune capacitive coupler is disclosed in US 6,956,450 B1. Here, the transmission line is not a strip line, but it is a low pass filter structure, suitable to suppress high frequency noise. It is terminated at its ends with the characteristic impedance of the line.

All large rotary joints such as the rotary joints used for CT scanners suffer from the problem, that they require a large rotating body or carrier for holding the transmission line, which must have a circular shape. Handling and transport of such a large body expensive and time consuming.

### Summary of the invention

The problem to be solved by the invention is to provide a rotary joint comprising a contactless data link, which comprises a plurality of sections. It should be a simple process to assemble the sections to a full body. Also disassembly should be possible without damaging of the body itself or further components thereon like the transmission line.

Solutions of the problem are described in the independent claims. The dependent claims relate to further improvements of the invention.

In an embodiment, the rotary joint body, holding the transmission line for a contactless data link, comprises multiple sections. The body may either have a disk shape or a drum shape. Preferably, the sections are arc segments or circular shaped segments. Preferably, the body is divided into two or four or any other number multiple of 2 segments, resulting in an even number, which most preferably have the same size. In the case of two segments, preferably each segment covers 180° of an arc, whereas in the case of four segments, each segment covers 90° of an arc. Preferably, each body segment has a transmission line section, most preferably at its outer side or outer circumference. Each transmission line section has an RF signal connector at one end and a transmission line termination at the other opposing end. The connector may be a plug- or socket connector. It may also have any combination thereof or may be hermaphroditic. The transmission line termination terminates the transmission line with its characteristic impedance, such that signals fed through the RF signal connector into the transmission line propagate through the transmission line section and are absorbed in the transmission line termination, preferably without causing any reflections of the signal. Preferably, a transmission line section covers the whole arc section of the body segment. In the case of a 180° body segment, the transmission line section preferably covers an angle of 180°, whereas in the case of a 90° segment, the transmission line section covers an angle of 90°.

For interconnecting the body segments to a full body, preferably mechanical connecting plates are provided. Such mechanical connecting plates may be attached to neighboring body segments, for example by means of screws, holding them together. In addition, at least one carrier plate is provided for further holding neighboring body segments together. This carrier plate preferably is held by screws to neighboring body segments. Furthermore, the carrier plate may hold an electronic housing which may further comprise at least one driver for generating and/or amplifying signals to be fed into the transmission line sections.

Body segments may comprise a plurality of sub-segments fixed together.

Body segments or sub-segments may be glued, screwed, welded or soldered together.

Neighbored body segment sections are aligned such that the RF signal connectors of neighboring body segments are close together. Therefore, the connectors of an electronic housing may connect two transmission line sections of neighboring body segments at the same time. This kind of connection avoids mismatch in signal delays and other signal properties like amplitude or common mode rejection, which may be caused, if the same signals would be processed by separate driver circuits being distant from each other. At the end of the body segments opposite to the location of an electronic housing, there are only terminations which do normally not require any active electronic, and therefore no electronic housing. At these locations, anyway a carrier plate, but without electronic housing, may be provided for firmly holding the body segments together.

### Description of Drawings

In the following the invention will be described by way of example, without limitation of the general inventive concept, on examples of embodiment with reference to the drawings.
Figure 1 shows a body segment interconnection
Figure 2 shows a further body segment interconnection
Figure 3 shows a complete slipring body
Figure 4 shows a partial assembled slipring body
Figure 5 shows a simplified embodiment
Figure 6 shows schematically a CT (Computed Tomography) scanner gantry

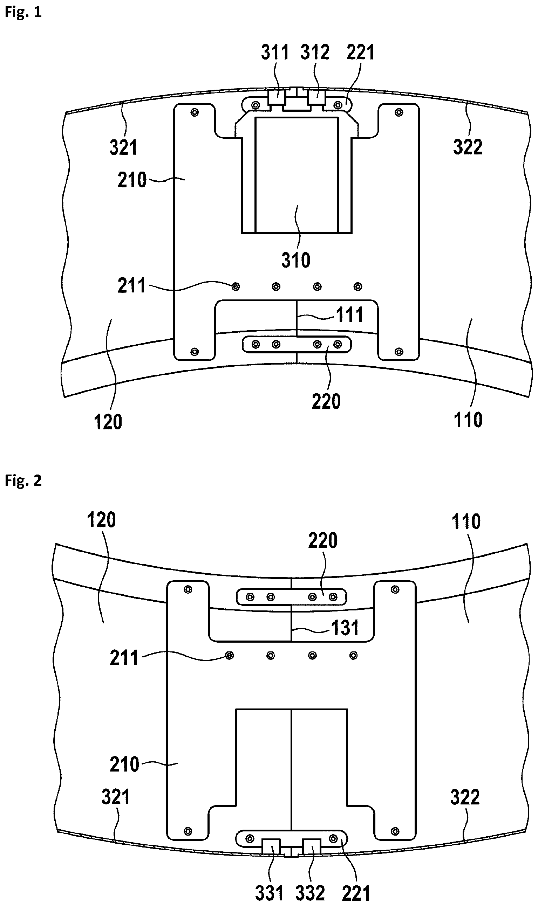

In figure 1 a partial view of a preferred embodiment is shown.

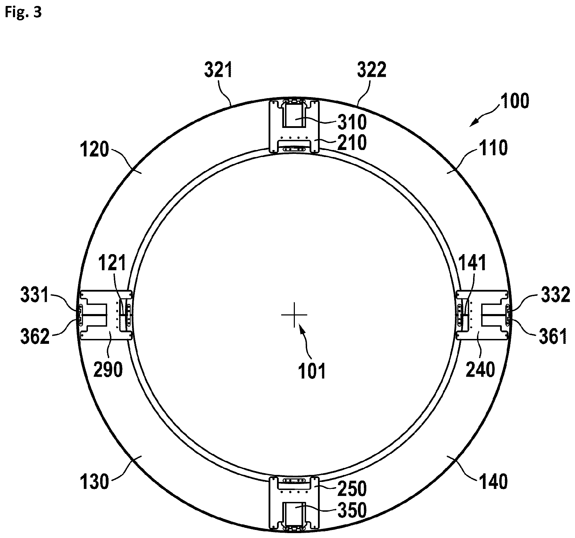

This Figure shows a partial view located at the intersection 111 between two neighbored body segments 110 and 120 as shown in Figure 3. The two neighbored body segments 110, 120 are mechanically held together by at least one connecting plate 220, 221. It is preferred to have at least an inner connecting plate 220 at the inner side of the body and an outer connecting plate 221 close to the outer side of the body. The connecting plates preferably are held by screws 211 to the body segments.

Furthermore, a carrier plate 210 is provided for firmly holding the body segments 110 and 120 together. This carrier plate preferably is held by screws 211 to the body segments. An electronic housing 310 is held by the carrier plate 210 at a predetermined position relative to the body segments. The electronic housing 310 preferably contains electronic devices like drivers for generating and/or amplifying transmission signals which are fed into transmission line sections 321, 322. Preferably, there are multiple transmission line sections at the outer circumference of the body. Most preferably, there are multiple of 2 such transmission line sections. In a most simple embodiment, there are only two such transmission line sections. In a more complex embodiment, there are four such transmission line sections, as will be shown later. As known from prior art, the signals to be coupled via the rotary joint are fed at one end into each transmission line section 321, 322 and propagate through the transmission line section, until they reach a termination 331, 332 at the end of the transmission line section.

The electronic housing respectively the driver circuit contained therein is connected via RF signal connectors 311, 312 to the transmission line sections. As shown herein, a first transmission line section 321 is located on second body segment 120, whereas second transmission line section 322 is located on first body segment 110. The first RF signal connector 311 for connecting the first transmission line section 321 is also located on second body segment 120. The second RF signal connector 312 for connecting the second transmission line section 322 is located on first body segment 110.

Preferably, each body segment has its own transmission line section together with its own RF signal connector. This allows for example an easy disassembly.

For disassembly of the embodiment as shown in this Figure, the attachment screws 211 of carrier plate 210 may to be removed first. Then, the electronic housing 310, which may still be connected to carrier plate 210, may be unplugged from the RF signal connectors 311, 312. Now, the transmission line sections 321 and 322 are separated from each other, and the body segments 110, 120 are only held together by connecting plates 220, 221 which may be removed later.

Assembly of this embodiment may also be done simply by first holding body segments 110 and 120 together by connecting plates 220, 221, which may be screwed into the body segments. In a next step, the electronic housing 310 together with attached carrier plate 210 is plugged into the RF signal connectors 311, 312 of transmission line sections 321, 322. Finally, carrier plate 210 is secured to the body segments 110, 120, firmly holding the body segments together.

Figure 2 shows a carrier plate 210 without electronic housing 310. Such carrier plates may be used alternating with carrier plates holding an electronic housing. Carrier plates without electronic housing may be used to hold body segments together, where transmission line terminations 331, 332 are located. Here also, connecting plates 220, 221 may be used.

Figure 3 shows a complete body 100 of the rotary joint. This exemplary embodiment comprises four body segments 110, 120, 130, 140 having intersections 111, 121, 131, 141. There is a first electronic housing 310 with first carrier plate 210 and opposing thereto a second electronic housing 350 with second carrier plate 250. The first electronic housing 310 with first carrier plate 210 is on the top and is connecting body segments 110, 120. The second electronic housing 350 with second carrier plate 250 is at the bottom, connecting body segments 130, 140. Between the carrier plates with electronic housing, at the locations of terminations 331, 332 there are carrier plates for termination, which hold no electronic housing. There is a first carrier plate for termination 240 and a second carrier plate for termination 290 opposing thereto. Furthermore, inner connecting plates 220, 221 may be attached at these locations as described in figure 1. The body 100 shown herein can easily be disassembled into four sections, or the body may easily be assembled from four sections. These four sections require far less space than the whole assembled body. Therefore, they can easier be manufactured and are further saving transport and handling costs.

Here, only electronic housings 310 are required at the top and bottom position. At the left and right positions, where the terminations are located, electronic housings are not required. Here, carrier plates 240, 290 (without electronic housings) may still be provided to hold the neighboring body segments together, if the connecting plates are not sufficient.

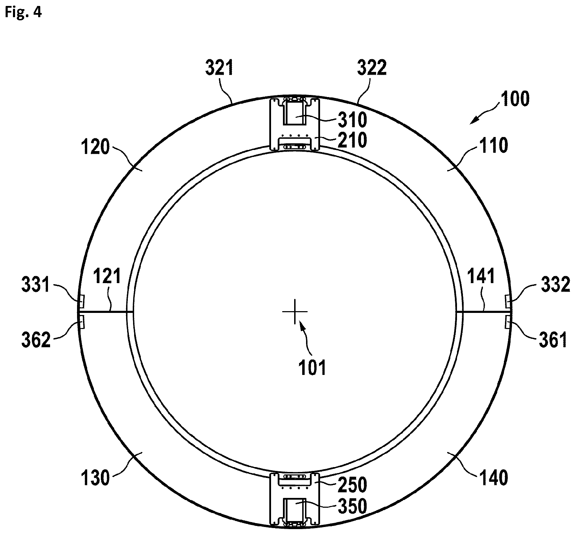

Figure 4 shows the embodiment of the previous figure partially assembled, without first and second first carrier plate for termination 240, 290. Here, the terminations for the transmission line sections can be seen. For example, first transmission line section 321 is terminated by first transmission line termination 331, and second transmission line section 322 is terminated by second transmission line termination 332. Third transmission line termination 361 and fourth transmission line termination 362 are provided for the transmission lines at the body segments 130, 140. These transmission lines are not shown in detail.

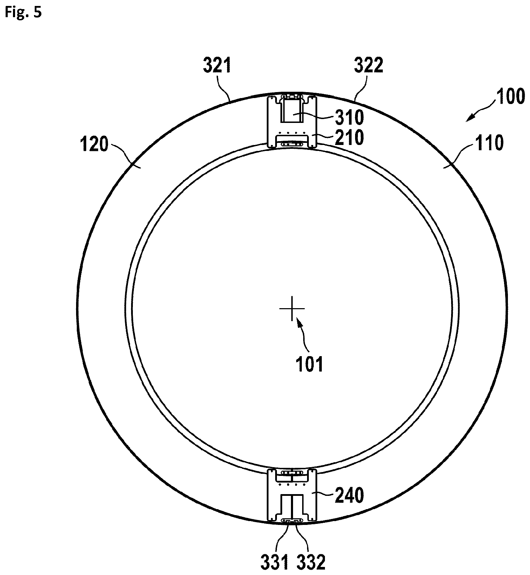

Figure 5 shows a simplified embodiment. This is similar to the embodiment of figure 3, but the body has only two body segments. Accordingly, only the first first carrier plate 210 with first electronic housing 310 and a first carrier plate for termination 240 are required.

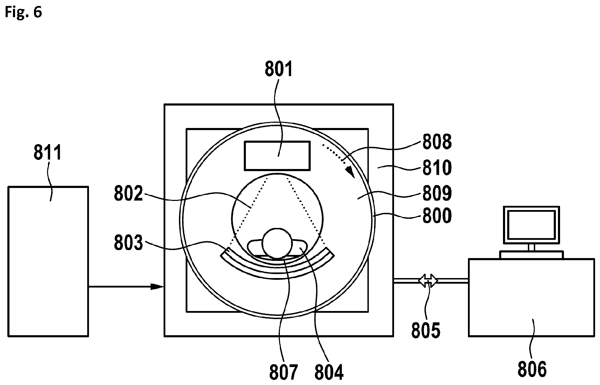

Figure 6 shows schematically a CT (Computed Tomography) scanner gantry. The stationary part is suspended within a massive frame 810. The rotating part 809 of the gantry is rotatably mounted with respect to the stationary part and rotates along the rotation direction 808. It supports an X - ray tube 801 for generating an X-ray beam 802 that radiates through a patient 804 lying on a table 807 and which is intercepted by a detector 803 and converted to electrical signals and imaging data thereof. Electrical power from power supply unit 811 may be transmitted by a slipring (not shown) to the rotating part. The data obtained by the detector 803 are transmitted via contactless rotary joint 800 to an evaluation unit 806 by means of a data bus or network 805.

### List of reference numerals

- 100: body
- 101: rotation axis
- 110, 120, 130, 140: body segments
- 111, 121, 131, 141: segment intersections
- 210: first carrier plate with housing
- 211: screws
- 220: inner connecting plate
- 221: outer connecting plate
- 240: first carrier plate for termination
- 250: second carrier plate with housing
- 290: second carrier plate for termination
- 310: first electronic housing
- 311: first RF signal connectors
- 312: second RF signal connector
- 321: first transmission line section
- 322: second transmission line section
- 331: first transmission line termination
- 332: second transmission line termination
- 350: second electronic housing
- 361: third transmission line termination
- 362: fourth transmission line termination
- 800: contactless rotary joint
- 801: x-ray tube
- 802: x-ray beam
- 803: x-ray detector
- 804: patient
- 805: network
- 806: evaluation unit
- 807: patient table
- 808: rotation direction
- 809: rotating part
- 810: frame
- 811: power supply unit

## Claims

1. Rotary joint comprising a body (100) and at least one transmisson line (321, 322) for a contactless data link,
the body (100) comprising a plurality of body segments (110, 120, 130, 140),
each body segment (110, 120, 130, 140) comprises one transmisson line (321, 322), the transmisson line comprising one RF signal connector (311, 312) at one end and a termination (331, 332) at the opposing end,
the body segments are oriented such, that alternatingly in neighboring segments two RF signal connectors or two terminations are next to each other,
two neighboring body segments are connected by a carrier plate (210, 240, 250, 290), wherein
the carrier plate holds an electronic housing (310, 350) where two RF signal connectors are next to each other, the electronic housing (310, 350) having a connection to the RF signal connectors.

2. Rotary joint according to claim 1, wherein the body (100) comprising a plurality of an even number of body segments (110, 120, 130, 140).

3. Rotary joint according to claim 1 or 2, wherein the body comprises 2 or 8 segments.

4. Rotary joint according to any one of the preceding claims, wherein at least one carrier plate (210, 240, 250, 290) is fixed by screws (211) to the segments.

5. Rotary joint according to any one of the preceding claims, wherein further at least one inner connecting plate (220) and/or outer connecting plate (221) is provided.

## Patentansprüche

1. Drehübertrager umfassend einen Körper (100) und zumindest eine Übertragungsleitung (321, 322) für eine kontaktlose Datenverbindung,
der Körper (100) umfassend eine Vielzahl von Körpersegmenten (110 ,120, 130, 140), jedes Körpersegment (110 ,120, 130, 140) umfassend eine Übertragungsleitung (321, 322),
die Übertragungsleitung umfassend einen HF-Signalverbinder (311, 312) an einem Ende und einen Abschluss (331, 332) am gegenüberliegenden Ende,
die Körpersegmente so ausgerichtet, dass abwechselnd in benachbarten Segmenten zwei HF-Signalverbinder oder zwei Abschlüsse nebeneinander sind,
zwei benachbarte Körpersegmente durch eine Trägerplatte (210, 240, 250, 290) verbunden sind, wobei
die Trägerplatte ein Elektronik-Gehäuse (310, 350) hält, wo zwei HF-Signalverbinder nebeneinander sind, das Elektronik-Gehäuse (310, 350) eine Verbindung zu den HF-Signalverbindern aufweisend.

2. Drehübertrager gemäß Anspruch 1,
wobei der Körper (100) eine Vielzahl einer geraden Anzahl von Körpersegmenten (110, 120, 130, 140) umfasst.

3. Drehübertrager gemäß Anspruch 1 oder 2,
wobei der Körper 2 oder 8 Segmente umfasst.

4. Drehübertrager gemäß einem der vorhergehenden Ansprüche,
wobei die zumindest eine Trägerplatte (210, 240, 250, 290) durch Schrauben (211) an den Segmenten befestigt ist.

5. Drehübertrager gemäß einem der vorhergehenden Ansprüche,
wobei weiterhin zumindest eine innere Verbindungsplatte (220) und/oder äußere Verbindungsplatte (221) bereitgestellt ist.

## Revendications

1. Collecteur tournant comprenant un corps (100) et au moins une ligne de transmission (321, 322) pour une liaison de données sans contact,
le corps (100) comprenant une pluralité de segments de corps (110, 120, 130, 140),
chaque segment de corps (110, 120, 130, 140) comprend une ligne de transmission (321, 322), la ligne de transmission comprenant un connecteur de signal RF (311, 312) au niveau d'une extrémité et un terminaison (331, 332) au niveau de l'extrémité opposée,
les segments de corps sont orientés de sorte que, en alternance dans des segments voisins, deux connecteurs de signal RF ou deux raccordements soient près l'un de l'autre,
deux segments de corps voisins sont connectés par une plaque-support (210, 240, 250, 290), dans lequel
la plaque-support supporte un boîtier électronique (310, 350) où deux connecteurs de signal RF sont près l'un de l'autre, le boîtier électronique (310, 350) ayant une connexion avec les connecteurs de signal RF.

2. Collecteur tournant selon la revendication 1,
dans lequel le corps (100) comprenant une pluralité d'un nombre pair de segments de corps (110, 120, 130, 140) .

3. Collecteur tournant selon la revendication 1 ou 2, dans lequel le corps comprend 2 ou 8 segments.

4. Collecteur tournant selon l'une quelconque des revendications précédentes,
dans lequel au moins une plaque-support (210, 240, 250, 290) est fixée par des vis (211) aux segments.

5. Collecteur tournant selon l'une quelconque des revendications précédentes,
dans lequel en outre au moins une plaque de connexion intérieure (220) et/ou une plaque de connexion extérieure (221) sont prévues.
